# FASCICULE DE BREVET EUROPEEN

(11) **EP 1 931 286 B1**
(45) Date de publication et mention de la délivrance du brevet: **10.07.2013**
(21) Numéro de dépôt: 06777470.3
(22) Date de dépôt: 27.06.2006
(51) Int. Cl.: A61F 5/00

(54) **CEINTURE GASTRIQUE**
MAGENBAND
GASTRIC BELT

(30) Priorité: 27.06.2005 FR 0506490
(43) Date de publication de la demande: 18.06.2008
(73) Titulaire: Medical Innovation Developpement, 69760 Limonest (FR)
(72) Inventeur: DENIS, Pierre André, F-69100 Villeurbanne (FR)
(74) Mandataire: Cabinet Plasseraud
(86) Numéro de dépôt international: PCT/EP2006/063575
(87) Numéro de publication internationale: WO 2007/000448

(56) Documents cités:
- EP-A- 0 769 282
- WO-A-00/00108
- FR-A- 2 799 118
- GB-A- 771 019

## Description

L'invention concerne le domaine technique des dispositifs destinés à être implantés au niveau de la zone de raccordement, entre la partie abdominale de l'oesophage et l'estomac, afin de créer une restriction locale permettant un contrôle de la quantité d'aliments ingérés par le patient porteur du dispositif.

Une ceinture gastrique gonflable selon le préambule de la revendication 1 est connue du document WO-A-00/00108.

Afin d'assurer cette restriction locale, il est connu de mettre en oeuvre une ceinture ou anneau gastrique gonflable, tel que, par exemple, décrit par les demandes de brevets EP 0 769 282, FR 2 799 118.

Selon ces documents, la ceinture gastrique gonflable comprend un corps tubulaire plat allongé, en matière souple, qui est, en partie au moins, élastiquement déformable et qui définit une chambre étanche gonflable pour présenter une face de travail, destinée à être placée au contact de l'estomac, et un dos à l'opposé de la face de travail.

Afin de permettre une fermeture de la ceinture en anneau, cette dernière comprend, également, des moyens de liaison équipant les extrémités du corps tubulaire et permettant de fermer la ceinture gastrique sous la forme d'un anneau, la face de travail étant, bien entendu, orientée vers l'intérieur. Enfin, la ceinture gastrique comprend, également, un cathéter de gonflage raccordé, de façon étanche, à la chambre gonflable et destiné à être raccordé à des moyens de gonflage. Les moyens de gonflage peuvent être, par exemple, constitués par un boîtier pourvu d'une membrane auto-obturable qui peut être transpercée par une aiguille de seringue ou analogue, au moyen de laquelle une injection ou un prélèvement de fluide, tel que, par exemple mais non nécessairement, du sérum physiologique, peut être effectué pour contrôler le gonflement de la chambre et ainsi les dimensions de l'étranglement stomacal réalisé au moyen de la ceinture gastrique gonflable.

Or, si de telles bandes ont donné généralement satisfaction et ont permis après leur implantation, dans la plupart des cas, d'atteindre l'effet thérapeutique recherché, il a été observé, dans un nombre certes réduit de cas, des problèmes lors du réglage des dimensions de l'étranglement stomacal et, notamment, lors du dégonflage de la ceinture gastrique.

Il est donc apparu le besoin de disposer d'un nouveau type de ceinture gastrique gonflable qui soit à même de prévenir les risques de blocage du dégonflage de la ceinture gastrique, notamment lorsque cette dernière est susceptible de présenter des plis.

Afin d'atteindre cet objectif, l'invention concerne une ceinture gastrique gonflable comprenant :
■ un corps tubulaire allongé en matière souple qui est, en partie au moins, élastiquement déformable, qui définit une chambre étanche gonflable et qui présente un dos et une face de travail,
■ des moyens de liaison disposés en relation avec les deux extrémités du corps tubulaire et permettant de fermer la ceinture gastrique sous la forme d'un anneau, la face de travail étant disposée à l'intérieur de l'anneau,
■ et un cathéter de gonflage destiné à être raccordé à des moyens de gonflage et raccordé, de façon étanche, à la chambre gonflable via un orifice de gonflage aménagé dans le dos du corps tubulaire allongé.

Selon l'invention, la paroi interne de la chambre gonflable présente, à proximité de l'orifice de gonflage, au moins une protubérance destinée à empêcher, lors du dégonflage de la chambre, une obstruction de l'orifice de gonflage par la paroi de la chambre correspondant à la face de travail située en regard de l'orifice de gonflage.

La présence de la protubérance évite, de manière fort avantageuse, que la paroi de la chambre gonflable ne vienne s'appliquer contre l'orifice de gonflage et ne l'obture, notamment lors d'une aspiration du fluide contenu dans la chambre gonflable.

Selon une caractéristique de l'invention et afin de garantir plus encore l'efficacité du système anti-obturation de l'orifice de gonflage, la paroi interne de la chambre gonflable présente plusieurs protubérances placées à proximité de l'orifice de gonflage et à distance les uns des autres.

Dans une forme préférée de réalisation, la paroi interne de la chambre de gonflage présentera alors une série de protubérances réparties autour de l'orifice de gonflage, en étant situées à distance les unes des autres. Compte tenu de la souplesse des matériaux généralement utilisés pour la réalisation du corps de la ceinture gastrique, il a été constaté que l'efficacité du système anti-obturation est assurée de manière optimale lorsque deux protubérances voisines sont séparées d'une distance **d,** mesurée au niveau de leur base sur la paroi interne de la chambre de gonflage, qui vérifie la relation 0,75 **e** ≤ **d** ≤ 2 **e,** où **e** correspond à l'épaisseur de la paroi de la chambre gonflable formant la face de travail du corps. De manière préférée, la distance **d** sera choisie pour vérifier la relation 1,3 **e** ≤ **d** ≤ 1,6 **e**. En effet, en choisissant une distance inter protubérances comprise dans cette plage de valeur, il a été constaté que cela laisse, en cas de placage de la face de travail contre la région de l'orifice de gonflage, subsister, entre les protubérances, des canaux suffisamment importants pour permettre une bonne évacuation du fluide contenu dans la chambre gonflable et cela, notamment, lorsque ce fluide est du sérum physiologique.

Selon l'invention, la ou les protubérances sont susceptibles de présenter différentes formes et, selon une autre caractéristique préférée de l'invention, chaque protubérance est formée par un plot de forme tronconique.

Selon une autre caractéristique de l'invention, chaque protubérance présente une hauteur **h** mesurée par rapport à la paroi interne de la chambre de gonflage qui vérifie la relation 1 **e** ≤ **h** ≤ 1,5 **e** et, de manière préférée, la relation 1 **e** ≤ **h** ≤ 1,3 **e** où **e** correspond à l'épaisseur de la paroi de la chambre gonflable formant la face de travail du corps.

Selon encore une autre caractéristique de l'invention, afin d'éviter d'éventuels problèmes de dégonflage au niveau de plis qui seraient formés par la face de travail de la ceinture fermée, la face interne de la paroi de la chambre comprend au moins une rainure, de direction longitudinale, destinée à définir un canal interne de circulation du fluide de gonflage au niveau du ou des plis formés. Selon l'invention, la rainure longitudinale ne s'étend pas nécessairement sur toute la longueur de la chambre gonflable, mais sur une partie au moins de cette longueur et, par exemple mais non exclusivement, dans une région médiane de la chambre et sur une longueur supérieure ou égale à la moitié de la longueur de la chambre gonflable.

Selon une forme préférée mais non strictement nécessaire, la face interne de la paroi de la chambre gonflable comprend, au moins, une série de rainures longitudinales parallèles.

Selon l'invention, cette rainure ou cette série de rainures peut être réalisée en tout endroit de la paroi de la chambre gonflable. Toutefois, de manière préférée, la ou les rainures longitudinales seront aménagées sur la face interne de la chambre gonflable correspondant au dos de la ceinture.

De manière préférée mais non strictement nécessaire, la face interne de la chambre gonflable, correspondant au dos de la ceinture, comprend alors deux séries de rainures longitudinales parallèles, chaque série étant située à proximité d'un bord du dos de la ceinture.

Bien entendu, les différentes caractéristiques de l'invention, évoquées ci-dessus, peuvent être mises en oeuvre toutes ensembles ou en partie seulement, selon différentes combinaisons pour la réalisation d'une ceinture gastrique gonflable conforme à l'invention.

Par ailleurs, diverses autres caractéristiques de l'invention ressortent de la description ci-dessous effectuée en référence aux dessins annexés qui illustrent une forme préférée, mais non limitative, de réalisation d'une ceinture gastrique gonflable selon l'invention.

La **fig. 1** est une perspective dans un état de repos d'une ceinture gastrique selon l'invention.

La **fig. 2** est une coupe longitudinale selon le plan **II-II** de la **fig. 1****.**

La **fig. 3** est une coupe transversale selon la ligne **III-III** de la **fig. 2****.**

La **fig. 4** est une vue de la ceinture gastrique gonflable selon la **fig. 1** dans un état fermé en anneau et semi gonflé.

La **fig. 5** est une coupe partielle, selon la ligne **V-V** de la **fig. 2****.**

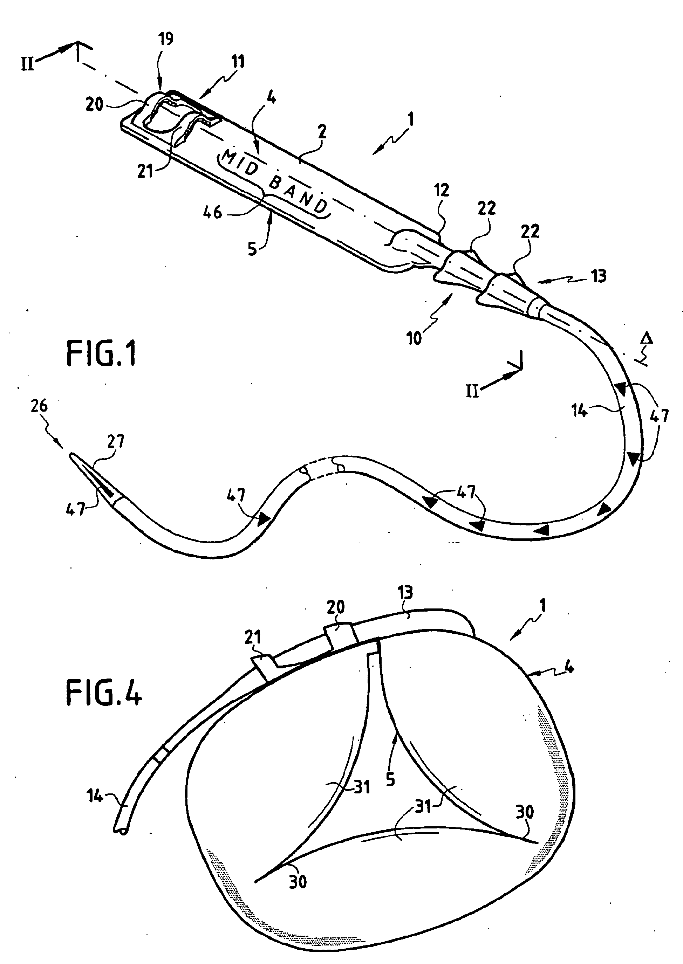

Une ceinture gastrique selon l'invention, illustrée aux **fig. 1** à **3** et désignée dans son ensemble par la référence **1,** comprend un corps tubulaire **2** qui définit au moins une chambre étanche gonflable **3.** Selon l'exemple illustré, le corps **2** présente, en élévation en vue de dessus, une forme générale rectangulaire, qui correspond à une forme préférée de réalisation, sans toutefois constituer la seule forme qui puisse être adoptée pour le corps **2.**

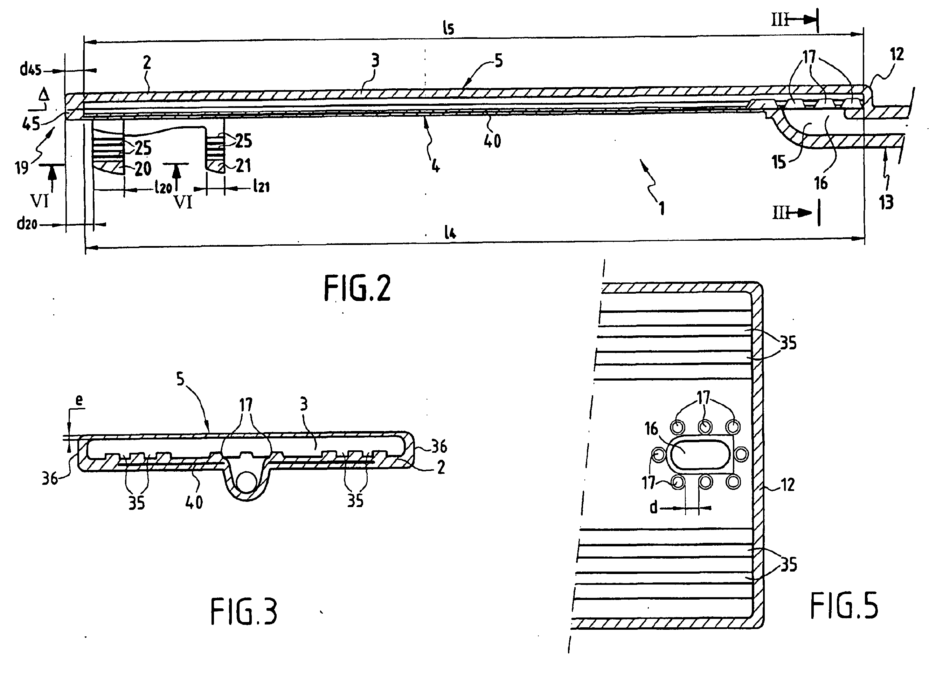

Il doit, par ailleurs, être considéré que, dans un état dégonflé de la chambre **3,** le corps **2** présente une forme générale plate et une section droite transversale, telle que plus particulièrement illustrée à la **fig. 3****,** sensiblement rectangulaire, la chambre gonflable **3** présentant une section droite transversale de forme générale s'inscrivant également dans un rectangle.

La ceinture gastrique **1** présente alors un dos **4** et une face de travail **5**, destinée à venir au contact de la zone oesophagienne ou stomacale, au niveau de laquelle la ceinture sera placée, comme cela ressortira de la suite.

Afin de permettre une fermeture de la ceinture **1** en anneau, comme cela est illustré à la **fig. 4****,** la ceinture **1** comprend, également, des moyens de liaison **10** et **11** équipant les deux extrémités de la ceinture. Les moyens de liaison **10** et **11** peuvent être réalisés de toute façon appropriée.

Selon l'exemple illustré, les moyens de liaison **10** comprennent, tout d'abord, au niveau d'une première extrémité **12** du corps **2,** dite de gonflage, une queue de raccordement **13** d'un cathéter **14** à la chambre gonflable **3.**

Selon l'exemple illustré, la queue de raccordement **13** s'étend alors sensiblement dans le prolongement longitudinal du corps **2** et présente un canal interne **15** raccordé à la chambre **3** par un orifice de gonflage **16**, comme le montre plus particulièrement la **fig. 5**. Bien entendu, ce canal **15** communique avec le conduit interne du cathéter **14** qui se trouve alors raccordé à la chambre **3,** via l'orifice de gonflage **16**.

Conformément à une caractéristique essentielle de l'invention, la paroi interne de la chambre de gonflage **3** présente alors au moins une et, selon l'exemple illustré, huit protubérances **17** qui, comme leur nom l'indique, s'étendent en saillie de la face interne de la chambre **3.** Ces protubérances **17** sont destinées à offrir un appui à la paroi de la chambre **3** constitutive de la face de travail **5** pour la maintenir à distance de l'orifice de gonflage **16** et éviter ainsi une obstruction de l'orifice de gonflage **16** par la paroi **5.** Afin d'aménager des passages de circulation vers l'orifice **16,** du fluide contenu dans la chambre **3** lorsque la paroi de la chambre est en appui sur les protubérances **17,** ces dernières sont distantes les unes des autres. De manière préférée, afin d'éviter que la paroi **5** ne puisse, compte tenu de sa souplesse, venir obstruer les interstices existants entre les protubérances **17** et cela tout en préservant des passages suffisamment importants pour le fluide de gonflage, les protubérances **17** sont disposées à une distance **d,** mesurée au niveau de leur base sur la paroi interne de la chambre de gonflage, qui vérifie la relation 0,75 **e** ≤ **d** ≤ 2 **e** et, de manière plus particulièrement, préférée la relation 1,3 **e** ≤ **d** ≤ 1,6 **e**, où **e** correspond à l'épaisseur de la paroi de la chambre gonflable formant la face de travail du corps. Dans le même sens, afin de garantir aux interstices existants entre les protubérances **17** une section de passage suffisante, les protubérances **17** sont, de manière préférée, réalisées pour présenter une hauteur **h** mesurée par rapport à la paroi interne de la chambre de gonflage qui vérifie la relation 1 **e** ≤ **h** ≤ 1,5 **e** et, de manière plus particulièrement préférée, la relation 1 **e** ≤ **h** ≤ 1,3 **e**. Il sera remarqué que, selon l'exemple illustré, les protubérances **17** possèdent une forme tronconique qui a été choisie en raison de sa facilité d'obtention, étant entendu qu'une autre forme pourrait être adoptée pour la ou les protubérances **17**.

Par ailleurs, les moyens de liaison **11**, situés au niveau de l'extrémité opposée à l'extrémité de gonflage **12** et dite extrémité libre **19**, sont, selon l'exemple illustré, constitués par au moins un et, dans le cas présent, de deux arceaux **20, 21** disposés au niveau du dos **4** du corps **2** et destinés à recevoir la queue **13**.

Selon l'exemple illustré, les deux arceaux **20** sont situés à distance l'un de l'autre et la queue **13** comprend des moyens **22** de blocage ou anti-retour destinés à empêcher tout retrait intempestif de la queue de raccordement **13** après engagement de cette dernière dans les arceaux **20, 21.** Selon l'exemple illustré, les moyens de blocage anti-retour comprennent deux conformations **22** en sapin ou lancéolées, qui sont chacune destinées à coopérer avec un arceau **20, 21** correspondant.

Selon l'exemple illustré, l'arceau **20,** situé le plus près de l'extrémité libre **19** du corps **2,** présente une largeur I₂₀, mesurée parallèlement à l'axe longitudinal Δ du corps **2** et, au sommet de l'arceau **20,** supérieure à 5 mm. Cette disposition de l'invention permet ainsi au premier arceau d'assurer un guidage du cathéter **14** lors de la procédure de fermeture de la ceinture. Selon l'exemple illustré, la largeur **I₂₀** du premier arceau est supérieure à la largeur I₂₁ du deuxième arceau.

De plus, toujours selon l'exemple illustré, afin de favoriser ce passage du cathéter et réduire, autant que faire se peut, les efforts nécessaires à ce geste chirurgical, les arceaux présentent, sur leur face interne, une série de stries **25** qui viennent réduire la surface de contact du cathéter avec l'arceau correspondant, de manière à réduire les forces de friction. Les stries **25** s'étendent parallèlement à l'axe longitudinal Δ du corps **2** et dans la direction d'introduction du cathéter **14.** Afin de réduire encore la force nécessaire au passage du cathéter **14,** il peut également être envisagé de recouvrir la surface extérieure du cathéter **14** d'un revêtement à faible coefficient de friction, tel que, par exemple, du téflon.

De même, l'extrémité libre **26** du cathéter **14** est obturée par un bouchon **27,** de forme conique, qui évite l'introduction de matières dans le canal du cathéter et facilite l'introduction du cathéter dans l'arceau **20** lors de la fermeture de la ceinture.

Le bouchon **27** sera sectionné après fermeture de la ceinture, pour la mise en place de moyens de gonflage, non représentés.

La ceinture gastrique gonflable **1** peut être réalisée en tout matériau biocompatible adapté, tel que, par exemple, du silicone biocompatible ou de grade implantable, qui confère au corps **2** la souplesse et l'élasticité nécessaire au gonflage de la chambre **3.**

Les moyens de liaison **10, 11** sont alors mis en oeuvre pour fermer en anneau la ceinture gonflable **1,** comme cela est illustré à la **fig. 4**.

Selon une forme préférée de l'invention, le corps **2** est réalisé de manière que, lors du gonflage de la chambre **3,** il se forme des plis **30** conférant, à la section de passage définie par la ceinture **1** en anneau, une forme d'étoile irrégulière ou, encore, d'hypocycloïde également irrégulière, comme le montre plus particulièrement la **fig.2****.**

Afin d'atteindre cet objectif recherché, de forme irrégulière ou aléatoire pour la section de passage définie par la ceinture gastrique **1** fermée en anneau et gonflée, le corps **2** est réalisé de manière que la longueur **I₅** de la paroi de la chambre gonflable **3,** formant la face de travail **5,** présente une longueur supérieure ou égale à la longueur **I₄** de la paroi de la chambre gonflable **3** formant le dos **4** de la ceinture **1** et cela dans un état dégonflé de ladite ceinture.

Selon l'exemple illustré, la longueur de la paroi **5** de la chambre gonflable 3 est sensiblement égale à celle de la paroi de la même chambre **3** constitutive du dos **4** de la bande de la ceinture gastrique **1.**

Selon la forme de réalisation illustrée, afin d'éviter des problèmes de dégonflage des différentes poches **31** formées par les plis **30**, la paroi interne de la chambre gonflable **3,** constitutive du dos **4,** présente deux séries de cannelures ou rainures de directions longitudinales **35** qui s'étendent, de préférence mais non nécessairement, sur toute la longueur de la paroi interne de la chambre gonflable **3.** Ces rainures **35** sont alors destinées à former des canaux au niveau des plis pour le passage du fluide de gonflage de la chambre **3.** Selon l'exemple illustré, les deux séries de rainures **35** sont placées à distance l'une de l'autre et chacune à proximité d'un bord longitudinal **36** du corps **2**.

Il doit être souligné que les plis **30**, réalisés par la ceinture gonflable **1** selon l'invention, contribuent à assurer une bonne stabilité de cette dernière au niveau de la paroi oesophagienne ou stomacale et limitent les mouvements de roulement susceptibles d'induire une inflammation de ladite paroi stomacale ou oesophagienne.

Par ailleurs, selon une autre caractéristique de l'invention, afin d'assurer un meilleur contrôle de la restriction stomacale formée par la ceinture **1**, il est également prévu d'incorporer, dans la paroi du corps **2** constitutif du dos **4,** une armature **40** souple, inextensible. Ainsi, lors du gonflage de la chambre **3,** la ceinture **1** connaît une déformation essentiellement centripète. L'armature souple **40** peut être réalisée en tout matériau souple inextensible adapté, tel que, par exemple mais non exclusivement, un tissu de dacron.

De manière préférée, l'armature **40** se trouve complètement noyée dans la paroi du corps **2** formant le dos **4** et se trouve complètement entourée du matériau constitutif du corps **2.**

Selon l'invention, afin de faciliter le travail du chirurgien utilisant la voie endoscopique, il peut être mis en oeuvre des moyens **46** de repérage optique du dos ou face dorsale **4** et/ou de la face de travail **5** du corps **2.** Selon l'exemple illustré **fig. 1****,** ces moyens de repérage **46** comprennent des caractères d'identification de la ceinture apposés sur le dos **4**, tandis que la face de travail est vierge. Ainsi, l'observation par l'endoscope de ces indications informe le chirurgien de l'orientation du corps de la ceinture. Selon l'exemple illustré, les indications **46** sont complétées par une série de marques **47,** aménagées sur la face du cathéter **14** correspondant au dos **4** du corps **2.** Les marques **47** présentent chacune ici la forme d'un triangle dont un sommet est dirigé vers l'extrémité et orienté vers l'extrémité libre **26** du cathéter, de sorte qu'elles remplissent également une fonction de moyen d'indication optique de la direction de l'extrémité libre du cathéter. L'observation des marques **47** facilite donc au chirurgien la mise en place de la ceinture.

Bien entendu, diverses modifications peuvent être apportées à l'invention sans sortir de son cadre.

## Revendications

1. Ceinture gastrique gonflable comprenant :
• un corps tubulaire allongé **(2)** en matière souple qui est, en partie au moins, élastiquement déformable, qui définit une chambre étanche gonflable **(3)** et qui présente un dos **(4)** et une face de travail **(5),**
• des moyens de liaison **(10, 11)** disposés en relation avec les deux extrémités **(12, 19)** du corps tubulaire **(2)** et permettant de fermer la ceinture gastrique sous la forme d'un anneau, la face de travail (5) étant disposée à l'intérieur de l'anneau,
• et un cathéter de gonflage destiné à être raccordé à des moyens de gonflage et raccordé, de façon étanche, à la chambre gonflable via un orifice de gonflage aménagé dans le dos du corps tubulaire allongé,
caractérisée
• en ce que la paroi interne de la chambre gonflable présente, à proximité de l'orifice de gonflage, au moins une protubérance destinée à empêcher, lors du dégonflage de la chambre, une obstruction de l'orifice de gonflage par la paroi de la chambre correspondant à la face de travail située en regard de l'orifice de gonflage,
• et en ce que, dans un état dégonflé de la chambre (3), le corps (2) présente une forme générale plate et une section droite transversale, sensiblement rectangulaire, la chambre gonflable (3) présentant une section droite transversale de forme générale s'inscrivant également dans un rectangle.

2. Ceinture gastrique gonflable selon la revendication 1, **caractérisée en ce que** la paroi interne de la chambre gonflable présente plusieurs protubérances placées à proximité de l'orifice de gonflage et à distance les uns des autres.

3. Ceinture gastrique gonflable selon la revendication 1 ou 2, **caractérisée en ce que** la paroi interne de la chambre de gonflage présente une série de protubérances réparties autour de l'orifice de gonflage en étant situées à distance les unes des autres.

4. Ceinture gastrique gonflable selon la revendication 2 ou 3, **caractérisée en ce que** deux protubérances voisines sont séparées d'une distance **d**, mesurée au niveau de leur base sur la paroi interne de la chambre de gonflage, qui vérifie la relation 0,75 **e** ≤ **d** ≤ 2 e et, de manière préférée, la relation 1,3 **e** ≤ **d** ≤ 1,6 **e**, où **e** correspond à l'épaisseur de la paroi de la chambre gonflable formant la face de travail du corps.

5. Ceinture gastrique gonflable selon l'une des revendications 1 à 4, **caractérisée en ce que** chaque protubérance est formée par un plot de forme tronconique.

6. Ceinture gastrique gonflable selon l'une des revendications 1 à 5, **caractérisée en ce que** chaque protubérance présente une hauteur **h** mesurée par rapport à la paroi interne de la chambre de gonflage qui vérifie la relation 1 **e** ≤ **h** ≤ 1,5 e et, de manière préférée, la relation 1 **e** ≤ **h** ≤ 1,3 **e,** où **e** correspond à l'épaisseur de la paroi de la chambre gonflable formant la face de travail du corps.

7. Ceinture gastrique gonflable selon l'une des revendications 1 à 6, **caractérisée en ce que** la face interne de la paroi de la chambre **(3)** comprend au moins une rainure **(35)** de direction longitudinale, destinée à définir un canal interne de circulation du fluide de gonflage au niveau des plis **(30)**.

8. Ceinture gastrique gonflable selon la revendication 7, **caractérisée en ce que** la face interne de la paroi de la chambre **(3)** comprend au moins une série de rainures longitudinales **(35)** parallèles.

9. Ceinture gastrique gonflable selon la revendication 7 ou 8, **caractérisée en ce que** la ou les rainures longitudinales sont aménagées sur la face interne de la chambre **(3)** correspondant au dos **(4)** de la ceinture.

10. Ceinture gastrique gonflable selon la revendication 7, **caractérisée en ce que** la face interne de la chambre, correspondant au dos de la ceinture, comprend deux séries de rainures longitudinales parallèles **(35),** chaque série étant située à proximité d'un bord **(36)** du dos **(4)** de la ceinture.

## Patentansprüche

1. Füllbares Magenband, umfassend:
- einen länglichen röhrenförmigen Körper (2) aus einem flexiblen Material, der zumindest teilweise elastisch verformbar ist, der eine füllbare dichte Kammer (3) definiert und der eine Rückseite (4) und eine Arbeitsseite (5) aufweist,
- Verbindungsmittel (10, 11), die in Verbindung mit den beiden Enden (12, 19) des röhrenförmigen Körpers (2) angeordnet sind und das Schließen des Magenbands in Form eines Rings ermöglichen, wobei die Arbeitsseite (5) im Inneren des Rings angeordnet ist,
- und einen Füllkatheter, der dafür bestimmt ist, mit Füllmitteln verbunden zu werden und über eine Füllöffnung, die in der Rückseite des länglichen röhrenförmigen Körpers vorgesehen ist, dicht mit der füllbaren Kammer verbunden zu werden,
**dadurch gekennzeichnet,**
- **dass** die Innenwand der füllbaren Kammer in der Nähe der Füllöffnung mindestens eine Ausstülpung aufweist, die dafür bestimmt ist, beim Füllen der Kammer das Versperren der Füllöffnung durch die Wand der Kammer, die der Arbeitsseite entspricht, die sich gegenüber der Füllöffnung befindet, zu verhindern,
- und dadurch, dass in einem nicht gefüllten Zustand der Kammer (3) der Körper (2) eine allgemeine flache Form und einen im Wesentlichen rechteckigen Querschnitt aufweist, wobei die füllbare Kammer (3) einen Querschnitt mit einer allgemeinen Form aufweist, die ebenfalls in einem Rechteck einbeschrieben ist.

2. Füllbares Magenband nach Anspruch 1, **dadurch gekennzeichnet, dass** die Innenwand der füllbaren Kammer mehrere Ausstülpungen aufweist, die in der Nähe der Füllöffnung und voneinander entfernt platziert sind.

3. Füllbares Magenband nach Anspruch 1 oder 2, **dadurch gekennzeichnet, dass** die Innenwand der Füllkammer eine Reihe von Ausstülpungen aufweist, die um die Füllöffnung verteilt und dabei voneinander entfernt befindlich sind.

4. Füllbares Magenband nach Anspruch 2 oder 3, **dadurch gekennzeichnet, dass** zwei benachbarte Ausstülpungen einen Abstand d zueinander aufweisen, der im Bereich ihrer Grundfläche an der Innenwand der Füllkammer gemessen ist, der die Beziehung 0,75 e ≤ d ≤ 2 e und vorzugsweise die Beziehung 1,3 e ≤ d ≤ 1,6 e erfüllt, wobei e der Dicke der Wand der füllbaren Kammer, die die Arbeitsseite des Körpers bildet, entspricht.

5. Füllbares Magenband nach einem der Ansprüche 1 bis 4, **dadurch gekennzeichnet, dass** jede Ausstülpung durch einen kegelstumpfförmigen Block gebildet ist.

6. Füllbares Magenband nach einem der Ansprüche 1 bis 5, **dadurch gekennzeichnet, dass** jede Ausstülpung eine Höhe h aufweist, die bezogen auf die Innenwand der Füllkammer gemessen ist, die die Beziehung 1 e ≤ h ≤ 1,5 e und vorzugsweise die Beziehung 1 e ≤ h ≤ 1,3 e erfüllt, wobei e der Dicke der Wand der füllbaren Kammer, die die Arbeitsseite des Körpers bildet, entspricht.

7. Füllbares Magenband nach einem der Ansprüche 1 bis 6, **dadurch gekennzeichnet, dass** die Innenseite der Wand der Kammer (3) mindestens eine Nut (35) in Längsrichtung umfasst, die dafür bestimmt ist, einen inneren Fließkanal für das Füllfluid im Bereich der Falten (30) zu definieren.

8. Füllbares Magenband nach Anspruch 7, **dadurch gekennzeichnet, dass** die Innenseite der Wand der Kammer (3) mindestens eine Reihe von parallelen Längsnuten (35) umfasst.

9. Füllbares Magenband nach Anspruch 7 oder 8, **dadurch gekennzeichnet, dass** die Längsnut(en) an der Innenseite der Kammer (3), die der Rückseite (4) des Bands entspricht, vorgesehen ist bzw. sind.

10. Füllbares Magenband nach Anspruch 7, **dadurch gekennzeichnet, dass** die Innenseite der Kammer, die der Rückseite des Bands entspricht, zwei Reihen von parallelen Längsnuten (35) umfasst, wobei sich jede Reihe in der Nähe eines Rands (36) der Rückseite (4) des Bands befindet.

## Claims

1. An inflatable gastric belt comprising:
■ an elongated tubular body (2) in a flexible material which at least in part is elastically deformable, which defines an inflatable sealed chamber (**3**) and which has a back (**4**) and a working face (**5**),
■ connecting means (**10, 11**) positioned in relationship with both ends (**12, 19**) of the tubular body (**2**) and with which the gastric belt may be closed as a ring, the working face (**5**) being positioned inside the ring,
■ and an inflation catheter intended to be connected to inflation means and sealably connected to the inflatable chamber via an inflation opening made in the back of the elongated tubular body,
characterized
• in that the inner wall of the inflatable chamber has, in proximity to the inflation opening, at least one protrusion intended to prevent, during deflation of the chamber, obstruction of the inflation opening by the wall of the chamber corresponding to the working face located facing the inflation opening,
• and in that, in a deflated condition of the chamber (**3)**, the body (**2)** has a general flat shape and a substantially rectangular transverse cross-section, the inflatable chamber (**3)** having a transverse cross-section with a general shape also inscribed in a rectangle.

2. The inflatable gastric belt according to claim 1, **characterized in that** the inner wall of the inflatable chamber has several protrusions placed in proximity to the inflation opening and at a distance from each other.

3. The inflatable gastric belt according to claim 1 or 2, **characterized in that** the inner wall of the inflation chamber has a series of protrusions distributed around the inflation opening while being located at a distance from each other.

4. The inflatable gastric belt according to claim 2 or 3, **characterized in that** two neighboring protrusions are separated by a distance **d**, measured at their base on the inner wall of the inflation chamber, which satisfies the relationship 0.75 **e** ≤ **d** ≤ 2 **e**, and, preferably, the relationship 1.3 **e** ≤ **d** ≤ 1.6 **e**, where **e** corresponds to the thickness of the wall of the inflatable chamber forming the working face of the body.

5. The inflatable gastric belt according to any of claims 1 to 4, **characterized in that** each protrusion is formed by a frusto-conical pin.

6. The inflatable gastric belt according to any of claims 1 to 5, **characterized in that** each protrusion has a height **h** measured relatively to the inner wall of the inflation chamber which satisfies the relationship 1 **e** ≤ **h** ≤ 1.5 **e**, and preferably the relationship 1 **e** ≤ **h** ≤ 1.3 **e**, where **e** corresponds to the thickness of the wall of the inflatable chamber forming the working face of the body.

7. The inflatable gastric belt according to any of claims 1 to 6, **characterized in that** the inner face of the wall of the chamber (**3**) comprises at least one groove (**35**) with a longitudinal direction, intended to define an inner channel for the flow of the inflation fluid at the folds (**30**).

8. The inflatable gastric belt according to claim 7, **characterized in that** the inner face of the wall of the chamber (**3**) comprises at least one series of parallel longitudinal grooves (**35**).

9. The inflatable gastric belt according to claim 7 or 8, **characterized in that** the longitudinal grooves are made in the inner face of the chamber (**3**) corresponding to the back (**4**) of the belt.

10. The inflatable gastric belt according to claim 7, **characterized in that** the inner face of the chamber, corresponding to the back of the belt, comprises two series of parallel longitudinal grooves (**35**), each series being located in proximity to an edge (**36**) of the back (**4**) of the belt.
